Europäisches Patentamt

European Patent Office

Office européen des brevets

(11) Veröffentlichungsnummer: **0 024 056**
**B1**

(12)

# EUROPÄISCHE PATENTSCHRIFT

(45) Veröffentlichungstag der Patentschrift:
27.11.85

(51) Int. Cl.⁴: **A 61 K 6/08**

(21) Anmeldenummer: **80104797.8**

(22) . Anmeldetag: **13.08.80**

(54) Verwendung eines Copolymeren aus Acrylsäure und Maleinsäure als Anmischkomponente für Glasionomerzemente.

(30) Priorität: **13.08.79 DE 2932823**

(43) Veröffentlichungstag der Anmeldung:
**18.02.81 Patentblatt 81/7**

(45) Bekanntmachung des Hinweises auf die Patenterteilung:
**27.11.85 Patentblatt 85/48**

(84) Benannte Vertragsstaaten:
**AT BE CH DE FR GB IT LI NL SE**

(56) Entgegenhaltungen:
**DE - A - 2 751 069**
**FR - A - 2 127 004**
**FR - A - 2 342 056**

Die Akte enthält technische Angaben, die nach dem
Eingang der Anmeldung eingereicht wurden und die
nicht in dieser Patentschrift enthalten sind.

(73) Patentinhaber: **Espe Fabrik Pharmazeutischer Präparate GmbH, D-8031 Seefeld / Obb (DE)**

(72) Erfinder: **Schmitt, Werner, Dr.chem., Prinzenweg 10, D-813 Starnberg (DE)**
Erfinder: **Purrmann, Robert, Dr.Chem., Riemerschmidstrasse 18, D-813 Starnberg (DE)**
Erfinder: **Jochum, Peter, Dr.Chem., Pointweg 5, D-8031 Hechendorf (DE)**
Erfinder: **Gasser, Oswald, Dr. Chem., Hartstrasse 13, D-8031 Seefeld (DE)**

(74) Vertreter: **Schübel-Hopf, Ursula et al, Strehl, Schübel-Hopf, Schulz Patentanwälte Widenmayerstrasse 17, D-8000 München 22 (DE)**

**Beschreibung**

Die Erfindung betrifft die Verwendung eines Maleinsäure-Acrylsäure-Copolymeren, gegebenenfalls mit üblichen Zusätzen, als Anmischkomponente für Glasionomerzemente.

In der Zahnheilkunde haben in neuerer Zeit Zemente auf Basis einer anorganischen pulverförmigen Komponente und eines Carboxylgruppen enthaltenden organischen Polymeren steigende Bedeutung als Befestigungszemente und zur Herstellung von Zahnfüllungen erlangt.

Die aus Zinkoxid und wässriger Polyacrylsäurelösung gebildeten Carboxylatzemente sind beispielsweise aus der DE-AS 1 617 688 bekannt. Sie sind zwar physiologisch unbedenklich, jedoch in kosmetischer Hinsicht wegen ihrer Opazität als sichtbares Dauerfüllungsmaterial ungeeignet. Darüber hinaus besitzen sie eine nur mässige mechanische Festigkeit. Vorteilhaft ist dagegen ihre chemische Bindung an Zahnschmelz und Dentin, so dass die Carboxylatzemente als Unterfüllungen und Befestigungszemente breite Anwendung gefunden haben. Aus dem tschechischen Urheberschein 156 804 ist bekannt, dass man die mechanische Festigkeit dieser aus Zinkoxid oder einem Gemisch aus Zinkoxid und Magnesiumoxid gebildeten Zemente verbessern kann, wenn man als Anmischkomponente eine wässrige Lösung eines Copolymeren der Acrylsäure mit 0,1 bis 50% einer anderen ungesättigten Carboxyverbindung oder deren Anhydrid verwendet. Die so gebildeten Zemente zeigen zwar verbesserte Festigkeit, ihre Nachteile in kosmetischer Hinsicht entsprechen jedoch denen des üblichen Zinkoxid-Polyacrylsäure-Systems.

Ein dentales Dauerfüllungsmaterial, das ausgezeichnete ästhetische Wirkung mit hoher Festigkeit verbindet, sind die sogenannten Silikatzemente, die aus einem Silikatglaspulver und einer wässrigen Lösung von Phosphorsäure gebildet werden.

Nachteilig an diesen Silikatzementen ist, dass sie pulpenschädlich und im Mundmilieu verhältnismässig löslich sind. Die zuerst genannte Eigenschaft ist besonders schwerwiegend; sie zwingt zur Anwendung von Kavitätenlacken oder Unterfüllungen, Verfahren, die zeitraubend und meist sehr unsicher sind.

Eine ausserordentliche Verbesserung gegenüber den vorstehend beschriebenen Silikatzementen wurde durch die sogenannten Glasionomerzemente erreicht, die nicht nur gute kosmetische und mechanische Eigenschaften besitzen, sondern auch in physiologischer Hinsicht befriedigen. Ein solches Zementsystem ist in der DE-OS 2 101 889 beschrieben. Die dort als Anmischflüssigkeit eingesetzten wässrigen Lösungen von Polymeren, die sich von ungesättigten α,β-Dicarbonsäuren mit 4 oder 5 Kohlenstoffatomen ableiten, führen zu einem Dentalzement, der ausgezeichnetes Abbindeverhalten, gute Festigkeit, gute physiologische Verträglichkeit und ästhetisches Aussehen besitzt.

Eine Übersicht über dieses Gebiet wird in dem Artikel von A. D. Wilson in «Chemical Society Reviews», 7 (2), Seiten 265 bis 296 (1978) gegeben.

Weitere Zementsysteme, deren Pulverkomponente aus einem pulverförmigen Metalloxid, wie Zinkoxid, oder einem Silikatzementpulver besteht, sind aus der DE-AS 2 439 882 bekannt.

Als Anrührflüssigkeit für diese Zemente werden wässrige Lösungen von Acrylsäure-Itaconsäure-Copolymeren verwendet, deren bevorzugtes Verhältnis von Acrylsäureeinheiten zu Itaconsäureeinheiten 19 : 1 bis 2 : 1 beträgt. Ferner werden in der GB-PS 1 382 881 Glasionomerzementsysteme beschrieben, deren Anmischkomponente aus einer wässrigen Lösung Copolymerer von ungesättigten α,β-Dicarbonsäuren, die mehr als 50 Mol-% der Einheiten der ungesättigten α,β-Dicarbonsäuren enthalten, besteht. Speziell in dieser Patentschrift genannte Polymere sind Maleinsäure-Itaconsäure-Copolymere sowie Copolymere der Maleinsäure mit Acryl- oder Methacrylsäure, die mehr als 90% Maleinsäure-Einheiten enthalten. Trotz ihrer vorstehend beschriebenen Vorteile in kosmetischer und physiologischer Hinsicht sind diese Zemente verbesserungsbedürftig, da ihre Haftung am Zahnschmelz und Zahnbein nicht zufriedenstellend ist. Diese Haftung könnte zwar durch Anwendung einer wässrigen Polyacrylsäurelösung als Anmischkomponente erreicht werden, in diesem Fall geht aber die günstige Abbindecharakteristik der Zemente verloren.

In der DE-OS 2 751 069 werden Polycarboxylatzemente beschrieben, die als Bestandteile der festen Komponente Zinkoxid und Silicumdioxid enthalten. Die organische Mischungskomponente dieser Polycarboxylatzemente ist ein Polymeres der Acrylsäure oder ein Copolymeres der Acrylsäure mit Maleinsäure, Aconitsäure, Fumarsäure, Citraconsäure, Mesaconsäure, Itaconsäure oder Methacrylsäure. Die im einzelnen in den Beispielen dieser Patentanmeldung beschriebenen Kombinationen aus einer Zinkoxid enthaltenden Pulverkomponente und einer organischen Mischungskomponente, die aus einem Copolymeren der Acrylsäure besteht, zeigen gegenüber Carboxylatzementsystemen, die unter Verwendung von Homopolymeren der Acrylsäure als Mischungskomponente erhalten werden, keinerlei Verbesserung der Eigenschaften des Zements, speziell der Druckfestigkeit.

Der Erfindung liegt daher die Aufgabe zugrunde, eine Anmischkomponente für Glasionomerzemente zur Verfügung zu stellen, die zu einem Zement führt, der nicht nur, wie die bekannten Zemente, gute mechanische Festigkeit und vorteilhaftes Abbindeverhalten zeigt, in kosmetischer und ästhetischer Hinsicht befriedigt und physiologisch so unbedenklich ist, dass er ohne Unterfüllung und ähnliche Vorkehrungen leicht in den Zahn eingebracht werden kann, sondern der darüber hinaus ausgezeichnetes Haftvermögen am Zahnschmelz und Zahnbein besitzt.

Es konnte nun gefunden werden, dass diese Aufgabe gelöst werden kann, wenn als Anmischungskomponente für Glasionomerzemente eine wässrige Lösung eines Maleinsäure-Acrylsäure-Copolymeren mit spezifischer Zusammensetzung eingesetzt wird.

Gegenstand der Erfindung ist demnach die Verwendung eines Copolymeren aus Acrylsäure und Maleinsäure mit einem Gehalt an 20 bis 65 Mol-% Acrylsäureeinheiten und 80 bis 35 Mol-% Maleinsäureeinheiten in Form einer 20 bis 65 gew.-%igen wässrigen Lösung oder in Form eines festen Pulvers als Anmischkomponente zur Herstellung eines Glasionomer-Dentalzements.

Angesichts des Standes der Technik war es überraschend, dass Acrylsäure-Maleinsäure-Copolymere in dem vorstehend definierten Bereich der Zusammensetzung besonders erhöhtes Haftvermögen am Zahnbein zeigen. Verwendet man nämlich Polymaleinsäure als Anmischkomponente für Glasionomerzemente, so erhält man einen Zement, der unbefriedigendes Haftvermögen am Zahnbein besitzt. Die Verwendung einer wässrigen Lösung von Polyacrylsäure als Anmischkomponente für Silikatzemente führt zwar zu guter Haftfestigkeit, aber zu einer unbrauchbaren Aushärtecharakteristik des Systems. Versucht man nun, einen geringen Anteil an Maleinsäureeinheiten in das Acrylsäurepolymere einzubauen, so verschlechtert sich das Haftvermögen der gebildeten Zemente drastisch. Es war daher keinesfalls zu erwarten, dass durch Verwendung von Maleinsäure-Acrylsäure-Copolymeren in einem Bereich höherer Konzentration an Maleinsäureeinheiten ein Maximum der Haftfestigkeit am Zahnschmelz und am Zahnbein erzielt werden kann.

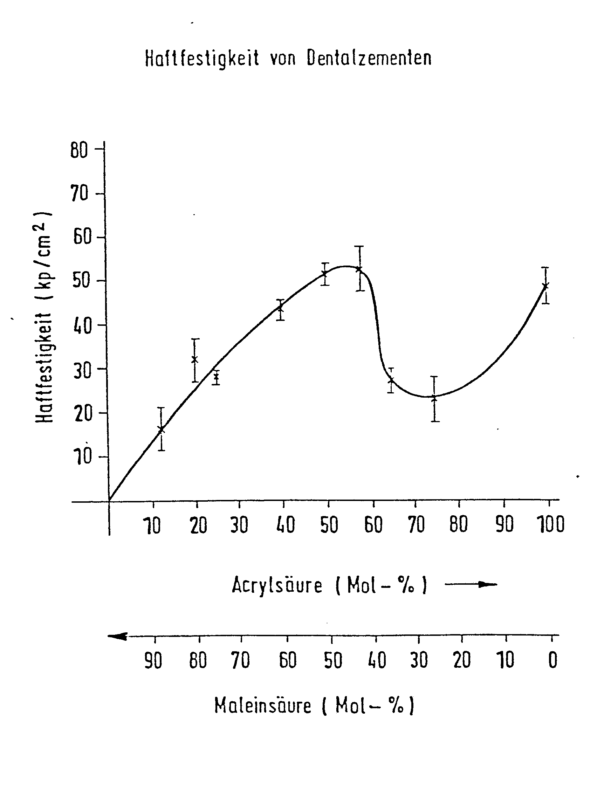

Die vorteilhafte Wirkung der erfindungsgemässen Anmischkomponente lässt sich durch die beigefügte Figur veranschaulichen, in der die Adhäsion in Abhängigkeit von der Zusammensetzung des Copolymeren aufgetragen ist.

Die Werte der Kurve wurden als durchschnittliche Mittelwerte von Reihenversuchen erhalten, nachdem die Prüfkörper eine Stunde lang bei 36°C und 100% Luftfeuchtigkeit und 20 Stunden unter Wasser bei 36°C gelagert worden waren. Aus der Kurve geht deutlich hervor, dass Maleinsäure-Acrylsäure-Copolymere mit geringem Gehalt an Acrylsäureeinheiten, jedoch auch mit hohem Gehalt an Acrylsäureeinheiten gegenüber reinen Acrylsäurehomopolymeren zu einer verschlechterten Haftfestigkeit führen, und dass ausschliesslich Copolymere im speziellen Bereich von 20 bis 65 Mol-% Acrylsäureeinheiten Systeme mit ausgezeichnetem Haftvermögen ergeben.

Besonders vorteilhaft für die Zwecke der Erfindung sind Copolymere, die etwa 40 bis 60 Mol-% Acrylsäureeinheiten und etwa 60 bis etwa 40 Mol-% Maleinsäureeinheiten enthalten.

Die Molverhältnisse sind stets als Durchschnittswert aller Moleküle des Copolymeren zu verstehen.

Gegenüber Anmischkomponenten, die Acrylsäure-Itaconsäure-Copolymere enthalten (DE-AS 2 439 882), zeigen die erfindungsgemässen Anmischkomponenten darüber hinaus eine deutlich verbesserte Druckfestigkeit, wie in dem später beschriebenen Vergleichsversuch gezeigt ist.

Die Herstellung der erfindungsgemäss eingesetzten Copolymeren ist an sich bekannt, sie wird beispielsweise in der DE-OS 2 212 623 (Beispiel 3) und in dem Artikel von A. A. El'Saied et al, Polym. Sci. USSR, 11, 314 (1969) beschrieben.

Das mittlere Molekulargewicht der geeigneten Copolymeren kann durch ihre Viskosität in wässriger Lösung gekennzeichnet werden. Bei einem Polymergehalt von 43% beträgt die Viskosität der wässrigen Lösung 0,5 bis 1000 P, vorzugsweise 1 bis 100 P und insbesondere 3 bis 50 P (jeweils bei 25°C).

Die Copolymeren der genannten Zusammensetzung können nach üblicher Reinigung für die Zwecke der Erfindung eingesetzt werden.

Obwohl grundsätzlich binäre Copolymere aus Acrylsäure- und Maleinsäureeinheiten eingesetzt werden, können die Copolymeren auch geringe Anteile an Einheiten zusätzlicher Comonomerer enthalten, solange die Eigenschaften des Copolymeren dadurch nicht verändert werden und das vorstehend definierte Molverhältnis von Maleinsäureeinheiten zu Acrylsäureeinheiten von 20 bis 65 molaren Anteilen Acrylsäureeinheiten auf 80 bis 35 molare Anteile Maleinsäureeinheiten eingehalten wird.

Die Pulverkomponente des erfindungsgemässen Zementsystems kann ein herkömmliches Silikatzementpulver sein, wie es in der DE-OS 2 101 889 beschrieben wird. Andererseits ist die erfindungsgemässe Anmischkomponente besonders vorteilhaft in Kombination mit Calciumaluminium-fluoro-silikatglaspulvern, die in der DE-OS 2 061 513 beschrieben sind. Geeignete Silikatzementpulver sind ausserdem in «Chemical Society Reviews», 7 (2), Seiten 265 bis 296 (1978) angegeben.

Die Dentalzemente werden gewöhnlich in Form eines Zweikomponentensystems angewendet, welches aus einer flüssigen Komponente und einer pulverförmigen Komponente besteht.

Die erfindungsgemässe Anmischkomponente kann gemäss einer Ausführungsform a) der Erfindung Teil der Anrührflüssigkeit und gemäss einer anderen Ausführungsform b) Teil der Pulverkomponente sein.

a) In dem zuerst genannten Fall besteht die Anrührflüssigkeit aus einer wässrigen Lösung des vorstehend definierten Maleinsäure-Acrylsäure-Copolymeren mit gegebenenfalls üblichen Zusätzen.

Das Copolymere liegt im allgemeinen in einer Konzentration von mindestens 20%, meist 30 bis 60 Gew.%, insbesondere 40 bis 50 Gew.% in der wässrigen Lösung vor, wenn das Zementsystem als Zahnfüllzement bestimmt ist. Für Befestigungszemente und prothetische Zwecke sind bereits niedrigere Konzentrationen vorteilhaft.

Die Lösungen der Copolymeren sollen Viskositäten von mindestens 0,5 Poise, jedoch höchstens 300 Poise haben. Ein bevorzugter Viskositätsbereich liegt zwischen 2 und 200, insbesondere zwischen 5 und 100 Poise/25°C.

Es ist üblich, Dentalpräparate prädosiert in sogenannten Schüttelkapseln zu vertreiben. In diesen werden Flüssigkeit und Pulver in zwei getrennten Kammern vorgesehen und unmittelbar vor Gebrauch vereinigt und mechanisch gemischt. Diese Prädosierung ist auch auf die erfindungsgemässe Anmischkomponente anwendbar.

b) Gemäss einer weiteren vorteilhaften Ausführungsform der Erfindung ist die Anmischkomponente, d.h. das Maleinsäure-Acrylsäure-Copolymere Bestandteil des Pulvergemisches und liegt als Vormischung aus Silikatzementpulver und Maleinsäure-Acrylsäure-Copolymerem vor. Die Pulverkomponente kann dann einfach mit Wasser, gegebenenfalls mit üblichen Zusätzen, angemischt werden.

In beiden Fällen können sowohl der Pulverkomponente als auch der flüssigen Komponente übliche Zusätze zugemischt werden, wie beispielsweise Chelatbildner, vorzugsweise Weinsäure, um die Aushärtecharakteristik zu verbessern (DE-OS 2 319 715). Der Weinsäurezusatz ist ohne Einfluss auf die Haftung des resultierenden Zements am Zahnschmelz. Zwischen beiden Ausführungsformen a) und b) sind fliessende Übergänge möglich: So kann zum Beispiel das erfindungsgemässe Copolymere je zur Hälfte der flüssigen und der pulverförmigen Anmischkomponente zugesetzt werden.

Auch bei der zuletzt genannten Ausführungsform b) kann es zweckmässig sein, sie prädosiert in Schüttelkapseln vorzusehen und zu vertreiben. Es kann auch besonders vorteilhaft sein, das Pulvergemisch zu einer Tablette zu verpressen.

Die Haftfestigkeit der Dentalzemente, die mit Hilfe der erfindungsgemässen Anmischkomponente erhalten wurden, wurde nach folgendem Verfahren gemessen:

Ein Rinderzahn wird plangeräst und die Schmelzschicht poliert und fettfrei gehalten. Der Zahn wird in Gips eingebettet und die präparierte Fläche planparallel zum Messtisch einjustiert. Das Calciumaluminium-fluoro-silikatglaspulver wird mit der ca. 44%igen wässrigen Lösung des entsprechenden Copolymeren im Masseverhältnis von etwa 3,0:1 angeteigt. Mit dieser Zementmischung wird ein Stahlzylinder von 30 mm Länge und 8 mm ∅ gefüllt. In 3 mm Abstand von der Einfüllöffnung wird durch Bohrungen ein Draht von 1 mm ∅ eingeführt, um eine geeignetere Retention zu schaffen.

Mittels einer Führung, die ein senkrechtes Aufsetzen gewährleistet, wird ein Stahlzylinder auf die plane Zahnfläche aufgebracht, und 10 min mit einer Masse von 100 g belastet.

Die Zähne mit den aufgesetzten Abzugskörpern werden folgendermassen gelagert:

10 min bei Raumtemperatur, 1 h bei 100% Luftfeuchtigkeit und 36°C, 20 h unter Wasser bei 36°C.

Nach der Lagerung wird der Stahlzylinder bei einem Vorschub von 4 mm pro Minute an einer Prüfmaschine der Firma Frank abgezogen. Die Kraft, die zum Abreissen des Stahlzylinders erforderlich ist, wird aufgezeichnet und ergibt nach der Multiplikation mit dem Faktor 34,3 die Adhäsion in Newton pro cm² (bzw. nach Multiplikation mit dem Faktor 3,5 die Adhäsion in Kilopond pro cm²). Die genannte Messung wird mit der gleichen Zementmischung an 5 verschiedenen Zähnen durchgeführt und daraus der Mittelwert gebildet.

Die mittels dieser Methode an erfindungsgemässen Materialien gemessenen Werte sind in der beigefügten Figur aufgetragen.

Die Messung der Viskosität der Copolymerlösungen erfolgte mit einem Rotationsviskosimeter. Anmeldungsgemäss wurde zu diesem Zweck das Gerät Haake Roto Visko mit den Drehkörpern HV I, Getriebezahl 3, Motordrehzahl 1 und der Eichkonstanten 48,5 verwendet. Durch Multiplikation des Skalenwerts mit diesen drei Parametern ergibt sich die absolute Viskosität in cP.

Die Erfindung wird durch die nachstehenden Beispiele näher erläutert. In diesen Beispielen sind allen Anmischflüssigkeiten 10 Gew.% Weinsäure zugemischt, soweit nichts anderes angegeben ist.

Beispiel 1

Ein für dentalmedizinische Zwecke geeignetes Acrylsäure-Maleinsäure-Copolymeres (Mol-Verhältnis 3:2) wird mit Wasser auf eine Konzentration von ca. 43 Gew.% gebracht. Die Lösung besitzt eine Viskosität von 12 P bei 25°C. Vermischt man diese Flüssigkeit mit handelsüblichem Silikatzementpulver (Syntrex) im Gewichtsverhältnis 1:2,5, so erhält man eine für die klinische Verwendung gut geeignete Paste, die in üblicher Weise in entsprechend vorbereitete Zahnkavitäten eingebracht wird. Das Gemisch erhärtet nach wenigen Minuten und weist in abgebundenem Zustand eine dem natürlichen Zahn entsprechende Transparenz auf. Die Füllung haftet gut an der gereinigten Zahnsubstanz, auch wenn auf die sonst üblichen mechanischen Retentionen verzichtet wird.

Beispiel 2

Aus einem Copolymeren mit einem Mol-Verhältnis Maleinsäure:Acrylsäure von 1:1 wird entsprechend Beispiel 1 eine Anmischflüssigkeit für Zahnzemente hergestellt. Ein handelsübliches Glasionomerzementpulver (G.C's Fuji) wird mit dieser Flüssigkeit im Gewichtsverhältnis 2,3:1 angeteigt. Der erhaltene Zement ist gut als Zahnfüllmasse geeignet. Die Haftkraft am Zahnschmelz beträgt 50 kp pro cm². Die Druckfestigkeit beträgt 1,860 kp pro cm².

Beispiel 3

Aus dem in Beispiel 2 genannten Copolymeren wird eine 35%ige wässrige Lösung hergestellt (Viskosität bei 25°C: 3 P). Nach Vermischen dieser Flüssigkeit mit käuflichem Glasionomerbefestigungszementpulver (De Trey Chembond) im Pulver-Flüssigkeitsverhältnis 1,2:1 erhält man einen dünnfliessenden Zement, der gute Haftung an der Zahnsubstanz zeigt und eine Druckfestigkeit von 1,100 kp pro cm² aufweist.

### Beispiel 4

In die Mischkammer von Schüttelkapseln, gemäss DE-OS 1 910 885, wird käufliches Glasionomerzementpulver (ASPA) in Portionen von 240 mg eingewogen. Als getrennte Kammer enthält diese Kapsel ein Folienkissen aus kunststoffkaschiertem Aluminium, das mit 96 mg der in Beispiel 1 beschriebenen Copolymerenlösung gefüllt ist. Wenn die so vorbereitete Kapsel wie in der oben genannten Offenlegungsschrift beschrieben, benutzt wird, erhält man nach dem Anmischen mit dem mechanischen Mischer einen Zement, der als Dauerfüllungsmaterial für Zahnkavitäten (besonders für Zahnhalsdefekte) geeignet ist und eine gute Klebkraft an der Zahnsubstanz aufweist.

### Beispiel 5

Ein trockenes, inniges Gemisch aus 300 mg käuflichem Glasionomerzementpulver (ASPA) und 41 mg des in Beispiel 2 erwähnten Copolymeren wird in üblicher Weise zu einer Tablette gepresst. Nach kurzem Anlösen dieser Tablette in 59 mg 14%iger Weinsäurelösung kann die Tablette leicht zu einer Zementpaste angemischt werden, die sich gut zur Füllung von Zahnhalsdefekten eignet und gute Haftfähigkeit an der Zahnsubstanz aufweist.

### Beispiel 6

Die in Beispiel 5 genannte trockene Pulvermischung (341 mg) wird in die Mischkammer einer Schüttelkapsel, gemäss DE-OS 1 910 885, gefüllt. Als getrennte Kammer enthält diese Kapsel ein Folienkissen aus kunststoffkaschiertem Aluminium, das mit 63 mg einer 15%igen wässrigen Weinsäurelösung gefüllt ist. Nach weiterer Anwendung, wie in Beispiel 4 beschrieben, wird ein an der Zahnsubstanz selbsthaftendes Füllungsmaterial von guter Druckfestigkeit erhalten.

### Vergleichsbeispiel

Dieser Versuch zeigt die erfindungsgemäss verbesserte Druckfestigkeit gegenüber einem bekannten Polycarboxylatzement auf Basis von Acrylsäure-Itaconsäure-Copolymeren.

Handelsübliches Glasionomerzementpulver (De Trey, ASPA) wurde mit der entsprechenden Anmischflüssigkeit im Pulver-Flüssigkeitsverhältnis 3,0:1 angeteigt. Aus der erhaltenen Zementmischung wurden in geeigneten Formen zylindrische Körper von 8 mm Länge und 4 mm ⌀ hergestellt. Die Körper wurden 24 Stunden bei 100% relativer Luftfeuchtigkeit gelagert und zur Ermittlung der Druckfestigkeit verwendet.

Ergebnisse:

| Anmischflüssigkeit | Druckfestigkeit |
|---|---|
| Acrylsäure-Itaconsäure-Copolymeres (De Trey, ASPA-Flüssigkeit) | 12 950 N/cm² (1320 kp/cm²) |
| Erfindungsgemäss, Acrylsäure-Maleinsäure-Copolymeres (Mol-Verhältnis 1 : 1) | 16 480 N/cm² (1680 kp/cm²) |

Gegenüber dem Stand der Technik ist die Druckfestigkeit mit den erfindungsgemässen Anmischflüssigkeiten um ca. ein Viertel höher.

### Patentansprüche

1. Verwendung eines Copolymeren aus Acrylsäure und Maleinsäure mit einem Gehalt an 20 bis 65 Mol-% Acrylsäureeinheiten und 80 bis 35 Mol-% Maleinsäureeinheiten in Form einer 20 bis 65 gew.%igen wässrigen Lösung oder in Form eines festen Pulvers als Anmischkomponente zur Herstellung eines Glasionomer-Dentalzements.

2. Verwendung nach Anspruch 1, dadurch gekennzeichnet, dass das Copolymere in Form einer wässrigen Lösung einer Konzentration von 30 bis 60 Gew.% vorliegt.

3. Verwendung nach Anspruch 1, dadurch gekennzeichnet, dass das Copolymere in einer Tablette vorliegt, die durch Verpressen eines Pulvergemisches aus dem Glasionomer-Zementpulver und dem Maleinsäure-Acrylsäure-Copolymeren sowie gegebenenfalls üblichen Zusätzen erhalten wurde.

4. Verwendung nach einem der Ansprüche 1 bis 3, dadurch gekennzeichnet, dass das Copolymere aus 40 bis 60 Mol-% Acrylsäureeinheiten und 60 bis 40 Mol-% Maleinsäureeinheiten besteht.

### Claims

1. Use of a copolymer of acrylic acid and maleic acid, containing 20 to 65 mole percent of acrylic acid units and 80 to 35 mole percent maleic acid units, either in the form of an aqueous solution having a concentration of 20 to 65 weight percent or in the form of a solid powder, as a mixing component for the production of a dental glass ionomer cement.

2. Use according to claim 1, characterized in that the copolymer is present in the form of an aqueous solution having a concentration of 30 to 60 percent by weight.

3. Use according to claim 1, characterized in that the copolymer is present in a tablet obtained by compressing a powder mixture of the glass ionomer cement powder and the maleic acid-acrylic acid-copolymer and optionally conventional additives.

4. Use according to any of the claims 1 to 3, characterized in that the copolymer consists of 40 to 60 mole percent of acrylic acid units and 60 to 40 mole percent of maleic acid units.

## Revendications

1. Application d'un copolymère d'acide acrylique et d'acide maléique avec une teneur en unités d'acide acrylique de 20 à 65 mole % et en unités d'acide maléique de 80 à 35 mole %, sous forme d'une solution aqueuse à 20–65% en poids, ou sous forme d'une poudre solide, comme composant de mélange, pour la préparation de ciments dentaires en ionomère de verre.

2. Application selon la revendication 1, caractérisée en ce que le copolymère se trouve sous la forme d'une solution aqueuse d'une concentration de 30 à 60% en poids.

3. Application selon la revendication 1, caractérisée en ce que le copolymère se trouve sous la forme d'une tablette qui a été obtenue par compression d'un mélange pulvérulent de poudre de ciment d'ionomère de verre et d'un copolymère acide maléique-acide acrylique, ainsi qu'éventuellement d'additifs usuels.

4. Application selon l'une des revendications 1 à 3, caractérisée en ce que le copolymère est constitué par 40 à 60 mole % d'unités d'acide acrylique et 60 à 40 mole % d'unités d'acide maléique.

Haftfestigkeit von Dentalzementen

Acrylsäure ( Mol – % ) ⟶

Maleinsäure ( Mol – % )